# EUROPEAN PATENT APPLICATION

(11) **EP 4 119 070 A1**
(43) Date of publication of application: **18.01.2023**
(21) Application number: 22182775.1
(22) Date of filing: 04.07.2022
(51) Int. Cl.: A61B 17/221, A61B 17/22

(54) **EXPANSILE FRAME ASSISTED VASCULAR OBSTRUCTION RETRIEVAL**

(30) Priority: 12.07.2021 US 202117372713
(71) Applicant: Neuravi Limited, Galway H91 K5YD (IE)
(72) Inventor: VALE, David, Galway, H91 K5YD (IE); CASEY, Brendan, Galway, H91 K5YD (IE); KEATING, Karl, Galway, H91 K5YD (IE); KELLY, Ronald, Galway, H91 K5YD (IE); MCCARTHY, Ray, Galway, H91 K5YD (IE); MIRZA, Mahmood, Galway, H91 K5YD (IE); JOHNSON, Sarah, Galway, H91 K5YD (IE); DWIVEDI, Anushree, Galway, H91 K5YD (IE)
(74) Representative: Carpmaels & Ransford LLP

(57) **Abstract**

Example systems and methods of treatment are presented herein which generally involve expanding a bracing frame within a blood vessel between a clot and an aspiration catheter, aspirating the clot through a passageway of the bracing frame, and supporting the blood vessel by the bracing frame as negative pressure is created by the aspiration. The bracing frame can thereby reduce likelihood of blood vessel collapse compared to a similar procedure which does not include the bracing frame. The bracing frame can be removed from the blood vessel during treatment. In some examples, the bracing frame can further function to expand a mouth of the catheter. In some examples, the system can include a stentriever.

## Description

### FIELD

The present invention generally relates devices and methods for removing acute blockages from blood vessels during intravascular medical treatments.

### BACKGROUND

Clot retrieval aspiration catheters and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular can be challenging because the target vessels are small in diameter, remote relative to the site of insertion, and highly tortuous.

In thrombectomy there is a tradeoff between deliverability of endovascular thrombectomy catheters and size of the device needed to capture the clot. Larger catheters are more difficult to deliver because they tend to be stiffer and less flexible. Clots are the same size or larger than a target vessel, thus catheters that are close to or the same size as the vessel are better able to capture the clot by complete ingestion. Further, once placed at a site, aspiration of a clot can cause blood vessels to collapse, preventing capture of the clot and potentially damaging the blood vessel.

### SUMMARY

Example systems and methods of treatment are presented herein which generally involve expanding a bracing frame within a blood vessel between a clot and an aspiration catheter, aspirating the clot through a passageway of the bracing frame, and supporting the blood vessel by the bracing frame as negative pressure is created by the aspiration. The bracing frame can thereby reduce likelihood of blood vessel collapse compared to a similar procedure which does not include the bracing frame. The bracing frame can be removed from the blood vessel during treatment. In some examples, the bracing frame can further function to expand a mouth of the catheter. In some examples, the system can include a stentriever. The stentriever is optional, can be delivered separately from the bracing frame in some examples, and can be delivered together with the bracing frame portion in some examples.

An example system configured to retrieve an obstruction from a blood vessel can include an aspiration catheter, a delivery catheter, and a tubular framework. The aspiration catheter can have a lumen sized to receive at least a portion of the obstruction therein. The aspiration catheter can be configured maintain an open flow path through the lumen of the aspiration catheter upon application of negative pressure due to aspiration through the lumen of the aspiration catheter. The delivery catheter can be sized to slide through the lumen of the aspiration catheter. The tubular framework can be collapsed within the delivery catheter. The tubular framework can be configured to expand to a deployed configuration within the blood vessel upon being expelled from the delivery catheter. In the deployed configuration the tubular framework can be configured to inhibiting radial collapse of the blood vessel due to the negative pressure in a negative pressure region of the blood vessel. In the deployed configuration the tubular framework can have a passageway therethrough sized to receive at least a portion of the obstruction therein.

The tubular framework can have a diameter of about 0.30 inches when collapsed within the delivery catheter.

The tubular framework can lack membrane cover. Alternatively, the tubular framework can include a membrane over a proximal portion of the tubular framework.

The aspiration catheter can have a malleable distal portion that is configured to expand in circumference upon expansion of a proximal portion of the tubular framework to the deployed configuration within the distal portion of the aspiration catheter.

The example system can further include a stentriever and a microcatheter configured to be delivered through the lumen of the aspiration catheter, through the passageway of the tubular framework while the tubular framework is in the deployed configuration, and across the obstruction. The stentriever can be configured to expand and engage the obstruction upon retraction of the microcatheter. At least a portion of the stentriever, when expanded, can be sized to enter the passageway of the tubular framework.

As an alternative, to the stentriever and microcatheter, the example system can include a clot retrieval device that includes the tubular framework and a stentriever portion. The clot retrieval device can include a bracing frame portion which includes the tubular framework and a collar. The stentriever portion can include an expandable framework and a pull wire. The collar of the bracing frame portion can circumscribe the pull wire. The clot retrieval device can be collapsed within the delivery catheter so that the tubular framework and stentriever can be delivered together. The bracing frame portion can further include a pull tube circumscribing the pull wire of the stentriever portion. The pull tube can be slidable over the pull wire to thereby move the expandable framework of the stentriever portion in relation to the tubular framework of the bracing frame portion. The pull wire can further include a radial extension that is positioned in a proximal direction in relation to the collar and that is configured to engage the collar of the bracing frame portion thereby inhibiting proximal movement of the bracing frame portion in relation to the stentriever portion.

A first example method of retrieving an obstruction from a blood vessel can include one or more of the follow steps executed in a variety of orders and combinations and including steps not described as understood by a person skilled in the pertinent art. A tubular framework can be expanded to a deployed configuration within the blood vessel such that in the deployed configuration at least a portion of the tubular framework is in contact with blood vessel walls in a proximal direction from the obstruction. An aspiration catheter can be positioned such that a mouth of the catheter is over a proximal portion of the tubular framework. A negative pressure region can be created within the blood vessel by aspirating through a lumen of the aspiration catheter while the tubular framework is in the deployed configuration. The blood vessel can be inhibited from collapsing radially in the negative pressure region by maintaining the tubular framework in the deployed configuration. At least a portion of the obstruction can be drawn into a lumen of the tubular framework by aspirating through the lumen of the aspiration catheter.

The first example method can further include sealing a membrane on the tubular framework to the blood vessel wall and to the mouth of the aspiration catheter.

The first example method can further include moving at least a portion of the tubular framework into the lumen of the aspiration catheter while at least a portion of the obstruction is within the lumen of the tubular framework.

The first example method can further include traversing the tubular framework and a bracing frame delivery catheter distally through the lumen of the aspiration catheter while the tubular framework is in a collapsed configuration within the bracing frame delivery catheter. The first example method can further include expelling the tubular framework from the bracing frame delivery catheter, thereby causing the tubular framework to move from the collapsed configuration to the deployed configuration.

The first example method can further include expanding a circumference of the mouth of the aspiration catheter when the tubular framework is expanded to the deployed configuration by providing a radially outward force from a proximal portion of the tubular framework against an inner wall of the lumen of the aspiration catheter approximate the mouth of the aspiration catheter.

The first example method can further include moving the mouth of the aspiration catheter distally over the proximal portion of the tubular framework while the tubular framework is in the deployed configuration to thereby expand the circumference of the mouth of the aspiration catheter.

The first example method can further include traversing a stentriever and a microcatheter distally through the lumen of the aspiration catheter, through the lumen of the tubular framework while the tubular framework is in the deployed configuration, and across the obstruction. The first example method can further include retracting the microcatheter, thereby causing the stentriever to expand across the obstruction. The first example method can further include pulling at least a portion of the stentriever and at least a portion of the obstruction within the lumen of the tubular framework while the tubular framework is in the deployed configuration.

The first example method can further include moving at least a portion of the tubular framework into the lumen of the aspiration catheter and at least a portion of the stentriever within the lumen of the aspiration catheter while at least a portion of the obstruction is within the lumen of the tubular framework.

The first example method can further include pulling a pull wire extending proximally from an expandable cage of the stentriever thereby pulling at least a portion of the stentriever and at least a portion of the obstruction within the lumen of the tubular framework. The first example method can further include engaging a radial extension of the pull wire to a collar of the tubular framework. The first example method can further include pulling the pull wire, thereby causing at least a portion of the tubular framework to move into the lumen of the aspiration catheter and at least a portion of the stentriever to move within the lumen of the aspiration catheter while at least a portion of the obstruction is within the lumen of the tubular framework.

The first example method can further include traversing a clot retrieval device and microcatheter through the lumen of the aspiration catheter while the clot retrieval device is in a collapsed configuration within the microcatheter. The clot retrieval device can include a bracing frame portion and a stentriever portion. The stentriever portion can include an expandable framework and a pull wire. The bracing frame portion can include the tubular framework and a collar. The collar can circumscribe the pull wire. The method can further include expelling the clot retrieval device from the microcatheter such that the expandable framework of the stentriever portion expands across the obstruction and the tubular framework expands to the deployed configuration.

The bracing frame portion can further include a pull tube circumscribing the pull wire of the stentriever portion. The first example method can further include sliding the pull tube in relation to the pull wire to thereby move the expandable framework of the stentriever portion in relation to the tubular framework of the bracing frame portion.

The first example method can further include engaging, to the collar of the bracing frame portion, a radial extension of the pull wire that is positioned in a proximal direction in relation to the collar, thereby inhibiting proximal movement of the bracing frame portion in relation to the stentriever portion.

A second example method of retrieving an obstruction from a blood vessel can include one or more of the following steps executed in a variety of orders and combinations and including steps not described as understood by a person skilled in the pertinent art. A tubular framework can be expanded to a deployed configuration within the blood vessel in a proximal direction in relation to the obstruction such that in the deployed configuration at least a portion of the tubular framework provides a radially outward force against blood vessel walls. A catheter can be positioned such that a mouth of the catheter is over a proximal portion of the tubular framework. A stentriever can be expanded across the obstruction. At least a portion of the stentriever and at least a portion of the obstruction can be pulled within a passageway through the tubular framework while the tubular framework is in the deployed configuration.

The second example method can further include sealing a membrane on the tubular framework to the blood vessel wall and to the mouth of the aspiration catheter.

The second example method can further include aspirating through a lumen of the catheter while the tubular framework is in the deployed configuration, thereby creating a negative pressure region within the blood vessel between the obstruction and the mouth of the catheter. The second example method can further include inhibiting radial collapse of the blood vessel in the negative pressure region by maintaining the tubular framework in the deployed configuration.

The second example method can further include moving at least a portion of the tubular framework into a lumen of the catheter and at least a portion of the stentriever within the lumen of the catheter while at least a portion of the obstruction is within the passageway through the tubular framework.

The second example method can further include pulling a pull wire extending proximally from an expandable cage of the stentriever thereby pulling at least a portion of the stentriever and at least a portion of the obstruction within the passageway through the tubular framework. The second example method can further include engaging a radial extension of the pull wire to a collar of the tubular framework. The second example method can further include pulling the pull wire, thereby causing at least a portion of the tubular framework to move into a lumen of the catheter and at least a portion of the stentriever to move within the lumen of the catheter while at least a portion of the obstruction is within the passageway through the tubular framework.

The second example method can further include traversing a clot retrieval device and microcatheter through a lumen of the catheter while the clot retrieval device is in a collapsed configuration within the microcatheter. The clot retrieval device can include a bracing frame portion and a stentriever portion. The stentriever portion can include an expandable framework and a pull wire. The bracing frame portion can include the tubular framework and a collar. The collar can circumscribe the pull wire. The second example method can further include expelling the clot retrieval device from the microcatheter such that the expandable framework of the stentriever portion expands across the obstruction and the tubular framework expands to the deployed configuration.

The bracing frame portion can further include a pull tube circumscribing the pull wire of the stentriever portion. The second example method can further include sliding the pull tube in relation to the pull wire to thereby move the expandable framework of the stentriever portion in relation to the tubular framework of the bracing frame portion.

The second example method can further include engaging, to the collar of the bracing frame portion, a radial extension of the pull wire that is positioned in a proximal direction in relation to the collar, thereby inhibiting proximal movement of the bracing frame portion in relation to the stentriever portion.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Figure 1 is an illustration of an example system during an example clot aspiration step according to aspects of the present invention.
Figures 2A through 2K are illustrations of example treatment steps including the example system and example step illustrated in Figure 1 according to aspects of the present invention.
Figures 3A through 3E are illustrations of example treatment steps that can be performed in place of steps illustrated in Figures 2H through 2K according to aspects of the present invention.
Figure 4 is an illustration of an example combination vascular device including a bracing frame portion and a stentriever portion according to aspects of the present invention.
Figures 5A and 5B are illustrations of alternative constructions of the combination vascular device illustrated in Figure 4 according to aspects of the present invention.
Figures 6A through 6D are illustrations of example treatment steps that can be performed using the combination vascular device illustrated in Figure 4 according to aspects of the present invention.
Figure 7 is an illustration of another example system during another example clot aspiration step according to aspects of the present invention.
Figure 8 is an illustration of another example system during another example clot aspiration step according to aspects of the present invention.

### DETAILED DESCRIPTION

When used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

For the sake of brevity, the term "clot" is used herein; however, it is to be understood that the systems and methods presented herein can be used to remove other types of obstructions from blood vessels.

When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

When used herein, the terms "pull wire" and "push tube" are to be construed broadly to include thin elongated structure that can each respectively be pulled and/or pushed to manipulate components of a related device and/or system. The terms "pull wire" and "push tube" can describe a structure having a solid core or a hollow core.

The example systems and methods of treatment described herein generally involve delivering a bracing frame device and an aspiration catheter, which are separate components, to a blood vessel obstruction (e.g. clot). A tubular framework of the bracing frame device can be expanded within a blood vessel between the obstruction and the aspiration catheter. The obstruction can be aspirated, by the aspiration catheter, through a passageway of the tubular framework. The tubular framework can support the blood vessel as negative pressure is created by the aspiration. The bracing frame device can thereby reduce likelihood of blood vessel collapse compared to a similar procedure which does not include the bracing frame device.

In some examples, the aspiration catheter can have a malleable distal portion, and the tubular framework can be expanded within the aspiration catheter to expand the malleable distal portion, thereby expanding a mouth opening of the aspiration catheter. Compared to a catheter having a self-expandable distal portion, the separate aspiration catheter and tubular framework may be made more simply and may be easier to deliver through vasculature. The tubular framework may or may not include a membrane or polymer jacket. Potential advantages of omitting the membrane may include improved deliverability, manufacturability, and cost effectiveness compared to a tubular framework having a membrane or polymer jacket. Regardless of whether the tubular framework includes a membrane or polymer jacket, or not, the separately deliverable catheter and tubular framework can provide improved deliverability, manufacturability, and cost effectiveness compared to a catheter having a built-in expandable frame at its distal end.

In some examples, the system can further include a stentriever that can expand through the obstruction and be retracted to draw the obstruction into the passageway of the tubular framework. The stentriever and tubular framework can be separate components or integral to a combination vascular device.

Various example systems and methods are presented herein. Features from each example are combinable with other examples as understood by persons skilled in the pertinent art.

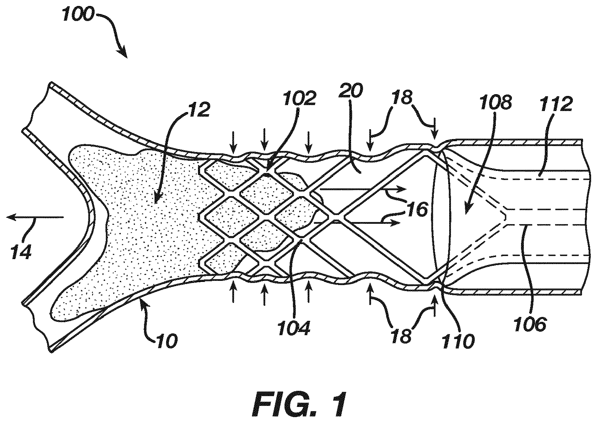

Figure 1 is an illustration of an example system 100 during an example clot aspiration step. The system 100 includes a bracing frame device 102 and an aspiration catheter 108 having an expandable mouth 110. The bracing frame device 102 includes a tubular framework 104 illustrated in a deployed configuration. During the illustrated step, negative pressure from aspiration 16 creates a force 18 on a portion of a blood vessel wall 10 within a negative pressure region 20 between the obstruction 12 and mouth 110 of the aspiration catheter 108. The tubular framework 104 of the bracing frame device 102 has a compressive hoop strength sufficient to inhibit collapse of the portion of the blood vessel wall surrounding the negative pressure region 20. The body 112 of the aspiration catheter 108 also has sufficient hoop strength to inhibit collapse of the aspiration catheter body 112 during aspiration.

A distal portion of the aspiration catheter 108, including the mouth 110, can be malleable. The malleable distal portion can be configured to expand in circumference due to a radially outward force applied from a proximal portion of the tubular framework 104 in the deployed configuration. As illustrated, a portion the bracing frame device 102 is expanded within the malleable distal portion of the aspiration catheter 108, thereby expanding the opening of the mouth 110. Because the malleable distal portion of the aspiration catheter 108 is supported by the expanded bracing frame device 102, the malleable distal portion need not have sufficient hoop strength to inhibit collapse of the distal portion. The malleability of the distal portion of the aspiration catheter 108 can result in the aspiration catheter 108 being easier to deliver compared to catheters having distal portions with high hoop strength, particularly expandable distal portions with high hoop strength.

Further, the mouth 110 can be expanded to contact walls of the blood vessel to create a seal between a perimeter of the mouth 110 and the blood vessel walls during aspiration. The tubular framework 104 need not include a membrane or cover to create a seal between the aspiration catheter 108 and the walls of the blood vessel 10.

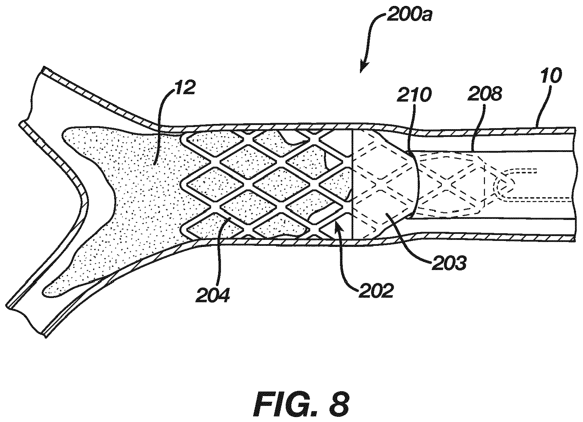

Although not illustrated as such in Figure 1, the tubular framework 104 can also be configured to dilate the blood vessel, expanding the blood vessel to a larger circumference than when the blood vessel has typical blood pressure similarly to as illustrated in Figure 8.

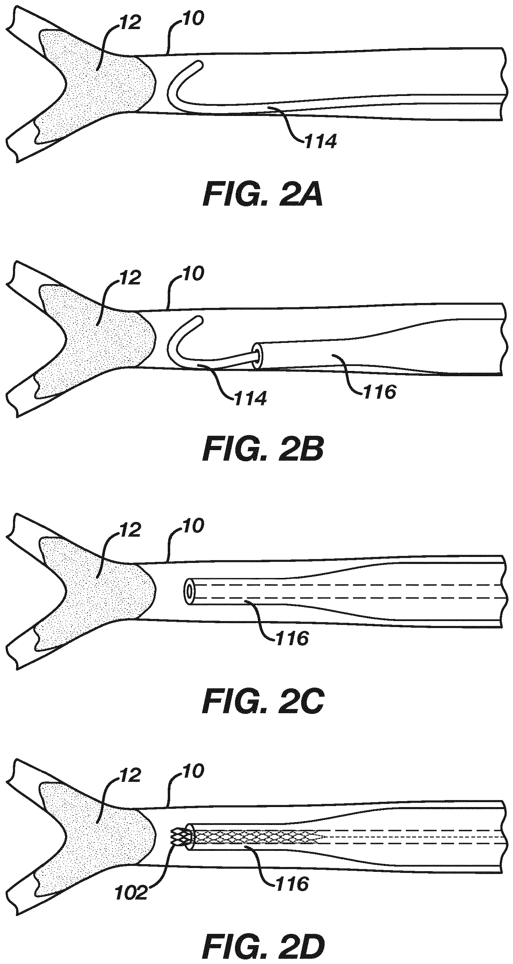

Figures 2A through 2K are illustrations of example treatment steps including the example system and example step illustrated in Figure 1.

Figure 2A illustrates a guide wire 114 positioned near, and in a proximal direction 22 from, a clot 12 at a bifurcation of a blood vessel 10.

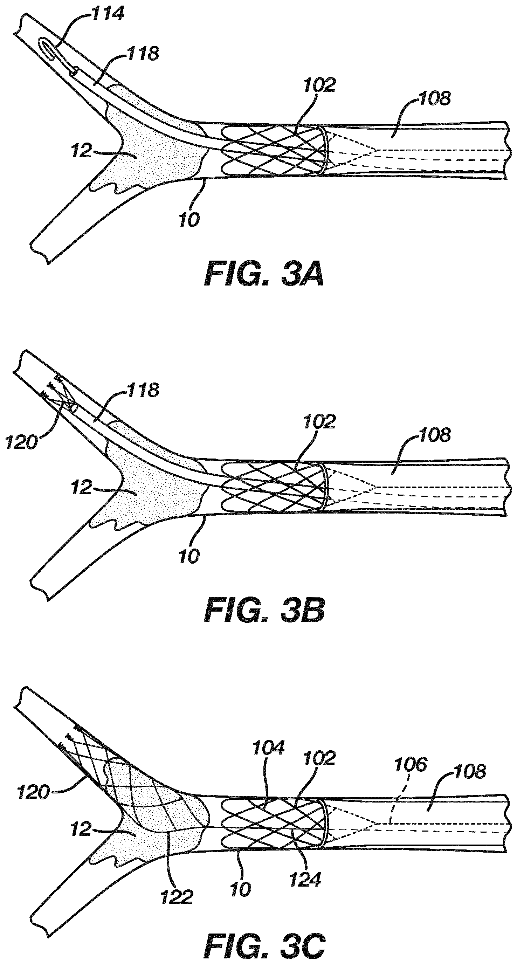

Figure 2B illustrates a delivery catheter 116 for the bracing frame device 102 delivered over the guide wire 114 near, and in the proximal direction 22 from the clot 12. As illustrated, the delivery catheter 116 has an enlarged outer diameter sized to assist delivery of the aspiration catheter 108 as described in greater detail in relation to Figure 2D. Alternatively, the delivery catheter 116 can be sized similar to the microcatheter 118 illustrated in Figures 3A and 3B.

Figure 2C illustrates the guide wire 114 withdrawn and the delivery catheter 116 remaining near the clot 12.

Figure 2D illustrates the bracing frame device 102 being delivered distally through the delivery catheter 116. In some examples, the bracing frame device 102 can collapse down to fit within a lumen having a small diameter of about 0.03 inches (about 0.8 millimeters). Struts of the tubular framework 104 can be nearly parallel to one another in the collapsed state. The parallel configuration of the tubular framework 104 can minimize the framework's tendency to expand and lock in the delivery catheter 116 during delivery. A tubular framework 104 lacking a membrane or cover can collapse to a smaller diameter than a comparable framework having a membrane or cover.

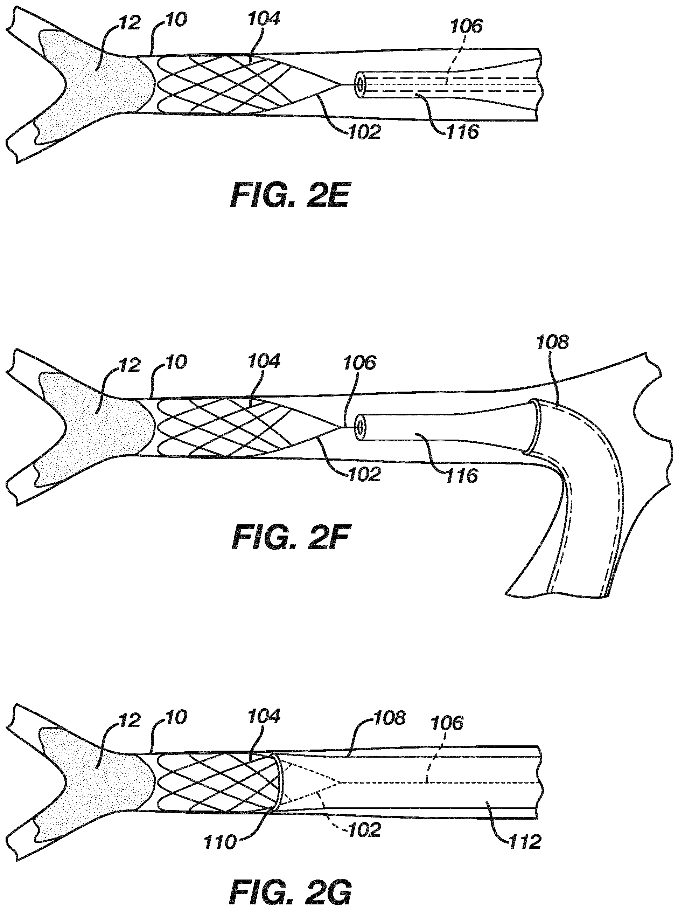

Figure 2E illustrates the delivery catheter 116 retracted proximally, thereby allowing the tubular framework 104 of the bracing frame device 102 to expand to walls of the blood vessel 10. In some treatments, the pull wire 106 of the bracing frame device 102 can be pulled in tension by pulling proximally on the pull wire 106 while the tubular framework 104 is expanded against the walls of the blood vessel 10. Tension on the pull wire 106 can create a stable "guide rail" over which the aspiration catheter 108 can be advanced. The tension preferably does not cause the tubular framework 104 to move. In some treatments, the tension can straighten the blood vessel 10 to a small extent in a proximal direction in relation to the tubular framework 104.

Figure 2F illustrates the aspiration catheter 108 being delivered distally over the delivery catheter 116. The delivery catheter 116 can act as an aid to help the aspiration catheter 108 navigate challenges in anatomy (e.g. the distal interior carotid artery region). Alternatively, the delivery catheter 116 can be retracted prior to delivery of the aspiration catheter 108 and the aspiration catheter 108 can be delivered over a pull wire 106 of the bracing frame device 102. In either case, the bracing frame device 102 can act as an anchor to assist the delivery of the aspiration catheter 108. In yet another alternative, the aspiration catheter 108 can be positioned prior to delivery of the bracing frame device 102 and the delivery catheter 116 and bracing frame device 102 can be moved distally through the aspiration catheter 108 toward the clot 12.

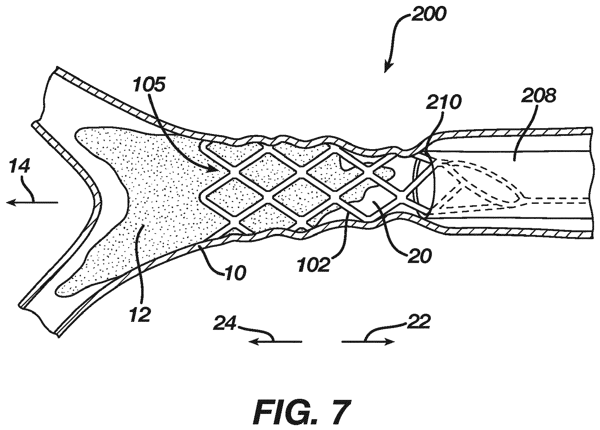

Figure 2G illustrates the aspiration catheter 108 being pushed distally over a proximal portion of the bracing frame device 102. If the catheter 108 includes a malleable distal portion as illustrated, the mouth 110 of the aspiration catheter 108 expands over the bracing frame device 102. If the distal portion of the catheter 208 is not malleable (see Figure 7), a proximal portion of the bracing frame device 102 can collapse into the mouth 210 of the catheter 208.

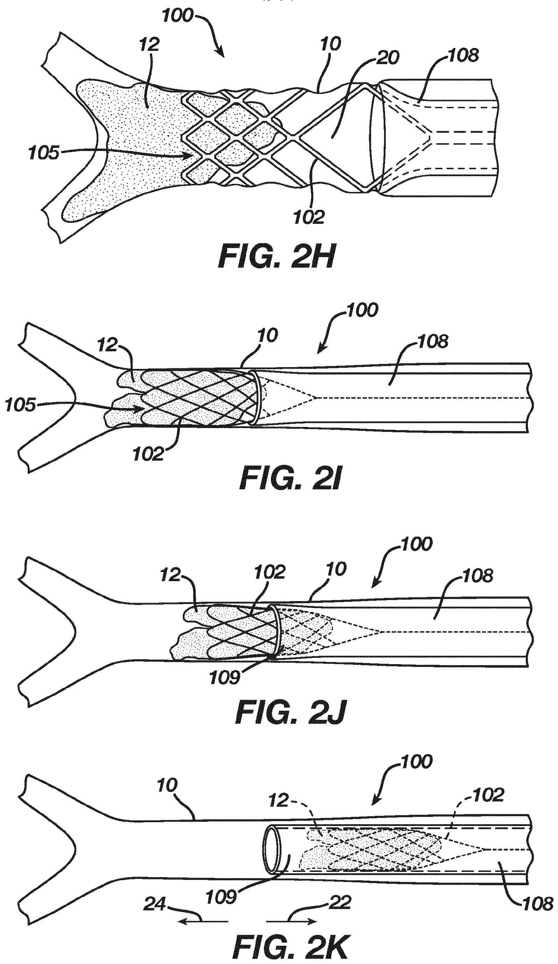

Figure 2H illustrates the aspiration step described in greater detail in relation to Figure 1. The aspiration causes the clot 12 to move into a passageway 105 of the tubular framework 104 of the bracing frame device 102.

Figure 2I illustrates the clot 12 moving further into the passageway 105 of the tubular framework 104 of the bracing frame device 102 due to aspiration.

Figure 2J illustrates the bracing frame device 102 being retracted in the proximal direction 22 into a lumen 109 of the aspiration catheter 108.

Figure 2K illustrates the tubular framework 104 and the clot 12 positioned entirely within the lumen 109 of the aspiration catheter 108. Preferably, the aspiration catheter 108 can remain positioned at the treatment site as the bracing frame device 102 and clot 12 are entirely removed from the aspiration catheter 108. In some treatments, the clot 12 may not fit entirely within the aspiration catheter 108 or the clot 12 may not be able to traverse the entire length of the aspiration catheter 108, in which case, the aspiration catheter 108 can be removed from a patient together with the clot 12 and bracing frame device 102.

Figures 3A through 3E are illustrations of example treatment steps that can be performed in place of steps illustrated in Figures 2H through 2K. The steps illustrated in Figures 3A through 3E illustrate a treatment utilizing a stentriever 120 separate from the bracing frame device 102.

Figure 3A illustrates the guide wire 114 with a microcatheter 118 thereover positioned across the clot 12. The guide wire 114 can be the same or different guide wire 114 illustrated in Figures 2A and 2B. Some treatments may not require the guide wire 114 at the illustrated step.

Figure 3B illustrates the guide wire 114 retracted and the stentriever 120 being expelled from a distal end of the microcatheter 118 in a distal direction 24 in relation to the clot 12.

Figure 3C illustrates the guide wire 114 and microcatheter 118 removed and the stentriever 120 expanded through the clot 12. Alternatively, the microcatheter 118 can be used to assist the stentriever 120 to engage the clot 12.

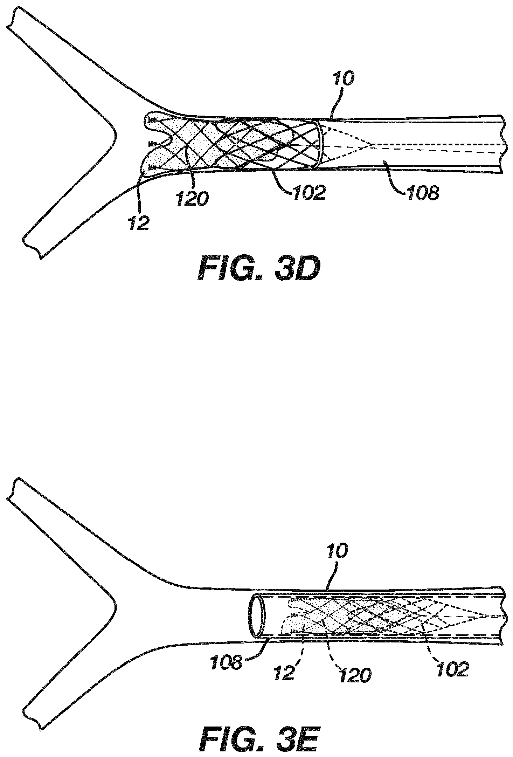

Figure 3D illustrates the stentriever 120 being pulled in the proximal direction 22 to move the clot 12 into the passageway 105 of the tubular framework 104 of the bracing frame device 102. In some treatments, it can be advantageous to aspirate during this step to assist in moving the clot 12.

Figure 3E illustrates a cage of the stentriever 120, the tubular framework 104, and the clot 12 positioned entirely within the lumen 109 of the larger catheter 108. Preferably, the aspiration catheter 108 can remain positioned at the treatment site as the stentriever 120, bracing frame device 102, and clot 12 are entirely removed from the aspiration catheter 108. In some treatments, the clot 12 may not fit entirely within the aspiration catheter 108 or the clot 12 may not be able to traverse the entire length of the aspiration catheter 108, in which case, the aspiration catheter 108 can be removed from a patient together with the clot 12, stentriever 120, and bracing frame device 102.

During the steps illustrated in Figures 3A through 3E, in some treatments it may be advantageous to aspirate in conjunction with mechanically retrieving the clot 12 with the stentriever 120, in which case the hoop strength of the bracing frame device 102 can be sufficient to inhibit collapse of the blood vessel 10 and the mouth 110 of the aspiration catheter 108. In some treatments, aspiration may not be advantageous, in which case the bracing frame device 102 can nevertheless provide an open passageway 105 through which to withdraw the clot 12 with the stentriever 120 and/or maintain a wide opening of the mouth 110 of the large catheter 108.

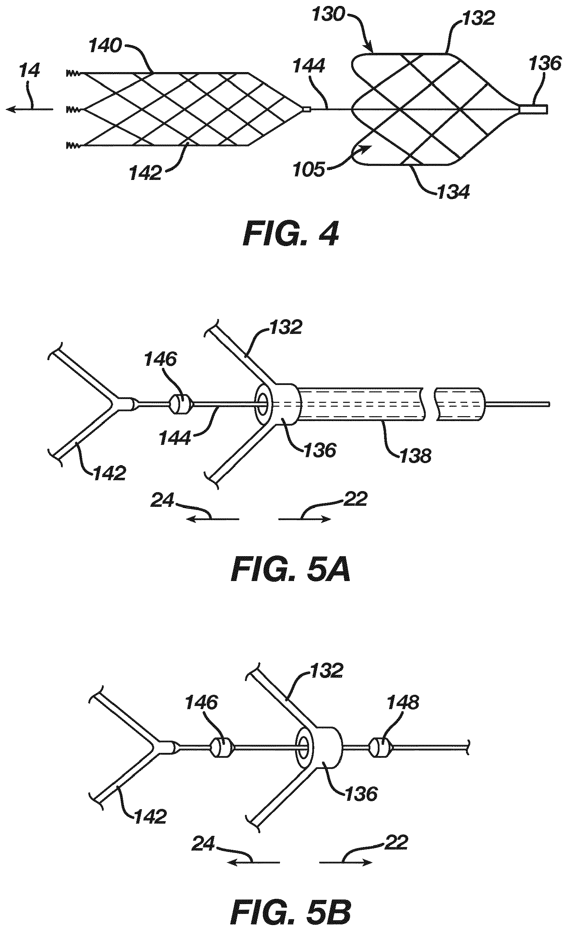

Figure 4 is an illustration of an example combination vascular device 130 including a bracing frame portion 132 and a stentriever portion 140. The stentriever portion 140 includes an expandable framework or cage 142 configured to mechanically engage the clot 12 and a pull wire 144 extending in the proximal direction 22 from the framework 142. The bracing frame portion 132 includes an expandable framework 134 that functions similarly to the tubular framework 104 of the bracing frame device 102 illustrated in the previous figures. The bracing frame portion 132 further includes a collar 136 that surrounds the pull wire 144 of the stentriever portion 140.

Figure 5A illustrates a first construction of the combination vascular device 130 that includes a distal radial extension 146 on the pull wire 144 of the stentriever portion 140. The distal radial extension 146 is positioned in the distal direction 24 in relation to the collar 136 of the bracing frame portion 132. The radial extension 146 is sized, positioned, and otherwise configured to engage the collar 136 of the bracing frame potion 132 to thereby inhibit distal movement of the bracing frame portion 132 in relation to the stentriever portion 140. The bracing frame portion 132 further includes a push tube, push wire 138 attached to the collar 136 and extending in the proximal direction 22 from the collar 136. The push tube 138 of the bracing frame portion 132 can be manipulated partially independent of the pull wire 144 of the stentriever portion 140.

During delivery and positioning of the bracing frame portion 132, the push tube 138 can be pushed in the distal direction 22 to move the bracing frame portion 132 distally. The push tube 138 can engage to the distal extension 146 so that pushing the push tube 138 also moves the stentriever portion 140 distally. Once the stentriever portion 140 is deployed, the push tube 138 can be pulled in the proximal direction to move the bracing frame portion 132 a desired distance from the stentriever portion 140.

Figure 5B illustrates a second construction of the combination vascular device 130 that includes a proximal radial extension 148 positioned in the proximal direction 22 in relation to the collar 136. The proximal radial extension 148 is sized, positioned, and otherwise configured to engage the collar 146 of the bracing frame portion 132 to thereby inhibit proximal movement of the bracing frame portion 132 in relation to the stentriever portion 140.

During delivery and positioning of the combination vascular device 130, the pull wire 144 of the stentriever portion 140 can be pushed in the distal direction 24, causing the proximal extension 148 to engage the collar 136 of the bracing frame portion 132, thereby moving the stentriever portion 140 and bracing frame portion 132 distally. The combination vascular device 130 is in a collapsed state, the pull wire 144 can be pushed distally until the bracing frame portion 132 is positioned as desired, then the pull wire 144 can be retracted in the proximal direction to move the stentriever toward the bracing frame portion 132 as desired.

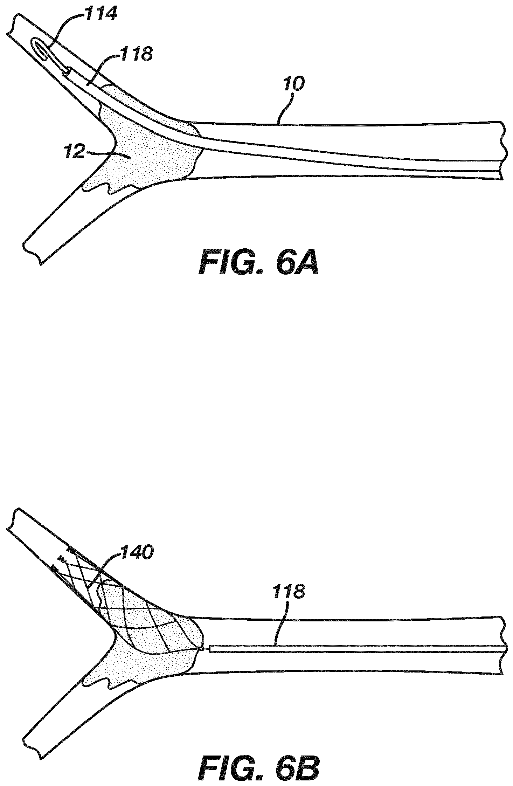

Figures 6A through 6D are illustrations of example treatment steps that can be performed using the combination vascular device illustrated in Figure 4.

Figure 6A illustrates a guide wire 114 and a microcatheter 118 positioned across the clot 12.

Figure 6B illustrates the microcatheter 118 retracted in the proximal direction 22 to allow the stentriever portion 140 of the combination vascular device 130 to expand into the clot 12.

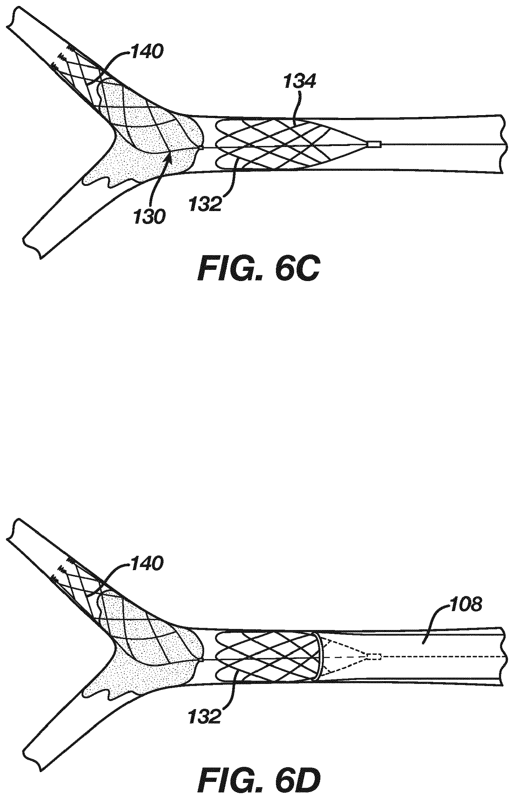

Figure 6C illustrates the microcatheter 118 further retracted in the proximal direction 22 to allow the tubular expandable framework 134 of the bracing frame portion 132 of the combination vascular device 130 to expand to walls of the blood vessel 10.

Figure 6D illustrates the aspiration catheter 108 being moved in the distal direction 24 over an expanded proximal portion of the bracing frame portion 132. Alternatively, the aspiration catheter 108 can be positioned prior to the delivery of the combination vascular device 130, in which case, the combination vascular device 130 and microcatheter 118 can be delivered through the lumen 109 of the aspiration catheter 108 while the combination vascular device 130 is in the collapsed configuration within the microcatheter 118.

Following the step illustrated in Figure 6D, the treatment can follow steps similar to those illustrated in Figure 3D and 3E.

Figure 7 is an illustration of another example system 200 during another example clot aspiration step. The system 200 illustrated in Figure 7 is similar to that illustrated in Figure 1 excepting that the aspiration catheter 208 illustrated in Figure 7 has a ridged distal portion, and as such, the mouth 210 does not expand when a portion of the bracing frame device 102 is expanding within the distal portion of the aspiration catheter 208. During the illustrated aspiration step, a portion of the blood vessel 10 in the negative pressure region 20 collapses to the tubular framework 104 of the bracing frame device 102. The partial collapse of the blood vessel 10 can cause the blood vessel to contact a perimeter of the mouth 210 of the aspiration catheter 208, thereby sealing the perimeter of the mouth 210 to the blood vessel wall. The aspiration catheter 208 illustrated in Figure 7 can be used in place of the aspiration catheter 108 in methods illustrated and described herein.

The system 200 illustrated in Figure 7 can be utilized following a similar method as illustrated in Figures 2A through 2K. In some treatments, the pull wire 106 of the bracing frame device 102 can be pulled in tension by pulling proximally on the pull wire 106 while the tubular framework 104 is expanded against the walls of the blood vessel 10. Tension on the pull wire 106 can create a stable "guide rail" over which the aspiration catheter 108 can be advanced. The tension preferably does not cause the tubular framework 104 to move. In some treatments, the tension can straighten the blood vessel 10 to a small extent in a proximal direction in relation to the tubular framework 104.

As an alternative to executing steps illustrated in Figures 2G through 2K, the aspiration catheter 208 can be pushed distally over the entirety of the tubular framework 104 and the bracing frame device 102 can be removed. In this alternative treatment, the bracing frame device 102 functions as a delivery assist device to deliver the aspiration catheter 108 and does not support the blood vessel 10 during aspiration.

Figure 8 is an illustration of another example system 200a during another example clot aspiration step. The system 200a illustrated in Figure 8 is similar to that illustrated in Figure 7 excepting that the tubular framework 204 includes a membrane 203 such that the tubular framework 204 and membrane 203 form a bracing frame device 202. The membrane forms a seal between walls of the blood vessel 10 and mouth 210 of the aspiration catheter 208. The membrane 203 can inhibit shrinkage of the blood vessel 10 near the mouth of the catheter 210 that is shown in Figure 7. The tubular framework 204 can further function to expand the blood vessel 10 as illustrated in Figure 8. The tubular framework 102 illustrated elsewhere can also be adapted to expand the blood vessel 10. The expandable framework 134 of the combination vascular device 130 illustrated in Figures 4 through 6D can be modified to include the membrane 203 illustrated in Figure 8.

The tubular frameworks 102, 134, 204 and stentriever structures 120, 140 presented herein are preferably laser cut from a biocompatible metal tube such as nitinol or other suitable material as understood by a person skilled in the pertinent art.

The descriptions contained herein are examples of embodiments of the invention and are not intended in any way to limit the scope of the invention. As described herein, the invention contemplates many variations and modifications of structures and methods, including alternative materials, alternative configurations of component parts, and alternative method steps. Modifications and variations apparent to those having skilled in the pertinent art according to the teachings of this disclosure are intended to be within the scope of the claims which follow.

### Aspects of the invention:

1. A method of retrieving an obstruction from a blood vessel, the method comprising:
   expanding a tubular framework to a deployed configuration within the blood vessel such that in the deployed configuration at least a portion of the tubular framework is in contact with blood vessel walls in a proximal direction from the obstruction;
   positioning an aspiration catheter such that a mouth of the catheter is over a proximal portion of the tubular framework;
   aspirating through a lumen of the aspiration catheter while the tubular framework is in the deployed configuration, thereby creating a negative pressure region within the blood vessel between the obstruction and the mouth of the aspiration catheter;
   inhibiting radial collapse of the blood vessel in the negative pressure region by maintaining the tubular framework in the deployed configuration; and
   drawing at least a portion of the obstruction into a passageway through the tubular framework by aspirating through the lumen of the aspiration catheter.
2. The method of aspect 1, further comprising:
   sealing a membrane on the tubular framework to the blood vessel wall and to the mouth of the aspiration catheter.
3. The method of aspect 1, further comprising:
   moving at least a portion of the tubular framework into the lumen of the aspiration catheter while at least a portion of the obstruction is within the passageway through the tubular framework.
4. The method of aspect 1, further comprising:
   traversing the tubular framework and a bracing frame delivery catheter distally through the lumen of the aspiration catheter while the tubular framework is in a collapsed configuration within the bracing frame delivery catheter; and
   expelling the tubular framework from the bracing frame delivery catheter, thereby causing the tubular framework to move from the collapsed configuration to the deployed configuration.
5. The method of aspect 1, further comprising:
   expanding a circumference of the mouth of the aspiration catheter when the tubular framework is expanded to the deployed configuration by providing a radially outward force from a proximal portion of the tubular framework against an inner wall of the lumen of the aspiration catheter approximate the mouth of the aspiration catheter.
6. The method of aspect 5, further comprising:
   moving the mouth of the aspiration catheter distally over the proximal portion of the tubular framework while the tubular framework is in the deployed configuration to thereby expand the circumference of the mouth of the aspiration catheter.
7. The method of aspect 1, further comprising:
   traversing a stentriever and a microcatheter distally through the lumen of the aspiration catheter, through the passageway through the tubular framework while the tubular framework is in the deployed configuration, and across the obstruction;
   retracting the microcatheter, thereby causing the stentriever to expand into the obstruction; and
   pulling at least a portion of the stentriever and at least a portion of the obstruction within the passageway through the tubular framework while the tubular framework is in the deployed configuration.
8. The method of aspect 7, further comprising:
   moving at least a portion of the tubular framework into the lumen of the aspiration catheter and at least a portion of the stentriever within the lumen of the aspiration catheter while at least a portion of the obstruction is within the passageway through the tubular framework;
   pulling a pull wire extending proximally from an expandable cage of the stentriever thereby pulling at least a portion of the stentriever and at least a portion of the obstruction within the lumen of the tubular framework;
   engaging a radial extension of the pull wire to a collar of the tubular framework; and
   pulling the pull wire, thereby causing at least a portion of the tubular framework to move into the lumen of the aspiration catheter and at least a portion of the stentriever to move within the lumen of the aspiration catheter while at least a portion of the obstruction is within the passageway through the tubular framework.
9. The method of aspect 8, further comprising:
   traversing a combination vascular device and microcatheter through the lumen of the aspiration catheter while the combination vascular device is in a collapsed configuration within the microcatheter,
      wherein the combination vascular device comprises a bracing frame portion and a stentriever portion,
      wherein the stentriever portion comprises an expandable framework and a pull wire,
      wherein the bracing frame portion comprises the tubular framework and a collar, and
      wherein the collar circumscribes the pull wire; and
   expelling the combination vascular device from the microcatheter such that the expandable framework of the stentriever portion expands into the obstruction and the tubular framework expands to the deployed configuration.
10. The method of aspect 9, wherein the bracing frame portion further comprises a pull tube circumscribing the pull wire of the stentriever portion, the method further comprising:
   sliding the pull tube in relation to the pull wire to thereby move the expandable framework of the stentriever portion in relation to the tubular framework of the bracing frame portion.
11. The method of aspect 9, further comprising:
   engaging, to the collar of the bracing frame portion, a radial extension of the pull wire that is positioned in a proximal direction in relation to the collar, thereby inhibiting proximal movement of the bracing frame portion in relation to the stentriever portion.

## Claims

1. A system configured to retrieve an obstruction from a blood vessel, the system comprising:
an aspiration catheter comprising a lumen sized to receive at least a portion of the obstruction therein;
a delivery catheter sized to slide through the lumen of the aspiration catheter; and
a tubular framework collapsed within the delivery catheter and configured to expand to a deployed configuration within the blood vessel in a proximal direction in relation to the obstruction upon being expelled from the delivery catheter such that in the deployed configuration the tubular framework is configured to inhibiting radial collapse of the blood vessel due to aspiration in a negative pressure region of the blood vessel and such that in the deployed configuration the tubular framework comprises a passageway therethrough sized to receive at least a portion of the obstruction therein.

2. The system of claim 1, wherein the tubular framework comprises a diameter of about 0.30 inches when collapsed within the delivery catheter.

3. The system of claim 1, wherein the tubular framework is uncovered.

4. The system of claim 1, wherein the tubular framework comprises a membrane over a proximal portion of the tubular framework.

5. The system of claim 1,
wherein the aspiration catheter comprises a malleable distal portion configured to expand in circumference due to a radially outward force applied from a proximal portion of the tubular framework in the deployed configuration.

6. The system of claim 1, further comprising:
a stentriever and a microcatheter configured to be delivered through the lumen of the aspiration catheter, through the passageway of the tubular framework while the tubular framework is in the deployed configuration, and across the obstruction,
wherein the stentriever is configured to expand and engage the obstruction upon retraction of the microcatheter, and
wherein at least a portion of the stentriever, when expanded, is sized to enter the passageway of the tubular framework.

7. The system of claim 1, further comprising:
a combination vascular device collapsed within the delivery catheter and comprising a bracing frame portion and a stentriever portion,
wherein the stentriever portion comprises an expandable framework and a pull wire,
wherein the bracing frame portion comprises the tubular framework and a collar,
wherein the collar circumscribes the pull wire, and
wherein the pull wire comprises a distal radial extension that is positioned in a distal direction in relation to the collar and that is configured to engage the collar of the bracing frame portion thereby inhibiting distal movement of the bracing frame portion in relation to the stentriever portion.

8. The system of claim 7, wherein the bracing frame portion further comprises a pull tube circumscribing the pull wire of the stentriever portion, the pull tube being slidable over the pull wire to thereby move the expandable framework of the stentriever portion in relation to the tubular framework of the bracing frame portion.

9. The system of claim 7, wherein the pull wire further comprises a radial extension that is positioned in a proximal direction in relation to the collar and that is configured to engage the collar of the bracing frame portion thereby inhibiting proximal movement of the bracing frame portion in relation to the stentriever portion.
